# EUROPÄISCHE PATENTSCHRIFT

(11) **EP 0 738 497 B1**
(45) Veröffentlichungstag und Bekanntmachung des Hinweises auf die Patenterteilung: **22.03.2000**
(21) Anmeldenummer: 95119680.7
(22) Anmeldetag: 14.12.1995
(51) Int. Cl.: A61B 6/14

(54) **Intraoraler Sensor zur Erstellung von Zahn-/Kieferaufnahmen eines Patienten**
Intraoral sensor for obtaining tooth/jaw images of a patient
Capteur intrabuccal pour obtenir des images des dents et de la mâchoire d'un patient

(30) Priorität: 22.04.1995 DE 29506839 U
(43) Veröffentlichungstag der Anmeldung: 23.10.1996
(73) Patentinhaber: Pfeiffer, Manfred, Dr., London W9 1EL (GB)
(72) Erfinder: Pfeiffer, Manfred, Dr., London W9 1EL (GB)
(74) Vertreter: Stenger, Watzke & Ring Patentanwälte

(56) Entgegenhaltungen:
- WO-A-92/22874
- WO-A-93/10709
- DE-U- 9 319 391
- DE-U- 9 419 116

## Beschreibung

Die Erfindung betrifft einen intraoral eines Patientenmundes plazierbaren Sensor zur Erstellung von Zahn-/Kieferaufnahmen eines Patienten, mit einem schmalen, in der Regel rechteckförmigen Gehäuse zur Aufnahme zumindest eines Bildsensors und einer diesem zugeordneten Steuerplatine, an der diverse Leitungen eines Anschlußkabels angeschlossen sind, welches an der Rückwand des Gehäuses aus dem Gehäuse austritt und mit einer Bildverarbeitungseinheit verbindbar ist.

Solche Intraoralsensoren sind beispielsweise aus den Druckschriften mit dem Titel "Visualix the new way to take dental X-rays" der Firma Philips Medical Systems und "SENS-A-RAY a revolution in dental radiography" der Firma "Regam Medical Systems AB" bekannt. Das Anschlußkabel tritt bei diesen bekannten Ausführungen entweder aus der schmäleren Stirnseite des Gehäuses oder rechtwinklig dazu an der Rückwand des Gehäuses aus.

Beide Ausführungsformen haben hinsichtlich der Positionierung des Sensors in der Mundhöhle des Patienten Nachteile, insbesondere wenn man Aufnahmen im Frontzahnbereich oder im hinteren Molarbereich erstellen möchte. Zum einen ist die Kabelführung für den Patienten unangenehm bzw. störend, wenn er den Sensor am aufzunehmenden Zahn positionieren will; zum anderen kann es leicht vorkommen, daß das aus dem Patientenmund herauszuführende Anschlußkabel unzulässig geknickt wird.

Die im wesentlichen selben Nachteile stellen sich bei einem Intraoralsensor ein, wie er in der Gebrauchsmusterschrift DE 93 19 391 U1 beschrieben ist. Das Anschlußkabel ist hierbei unter einem Winkel zwischen 30° und 60° zur Ebene der Rückwand aus dem Gehäuse herausgeführt, was von Patienten ebenfalls als unangenehm bzw. störend empfunden wird.

Der in Anspruch 1 angegebenen Erfindung liegt die **Aufgabe** zugrunde, demgegenüber eine Verbesserung zu schaffen.

Zur **Lösung** wird bei einem Intraoralsensor der eingangs genannten Art vorgeschlagen, daß das Kabel unter einem Winkel zwischen 0° und 10° zur Ebene der Rückwand aus dem Gehäuse austritt.

Mit einem solchen Intraoralsensor wird für nahezu alle Zahnaufnahmen eine optimale Positionierung des Sensors erzielt, ohne daß sich die Kabelführung für den Patienten unangenehm oder störend bemerkbar macht.

Vorteilhafterweise ist an der Kabelaustrittsstelle das Gehäuse mit einer konvexen Wölbung versehen, die durch eine am Gehäuse angeformte Kappe gebildet sein kann. Darin lassen sich sehr gut die Anschlußleitungen unterbringen, ohne daß das Gehäuse insgesamt baulich vergrößert werden muß. Die Wölbung dient zugleich als Positionierhilfe für den Patienten beim Anlegen des Sensors an den abzubildenden Zahn.

Von besonderem Vorteil ist es, wenn das Anschlußkabel im Bereich des Kabelaustritts von einer elastischen Versteifungsmanschette umgeben ist. Die bei der Anwendung des Intraoralsensors unvermeidlichen Biegungen des Anschlußkabels führen auf diese Weise auch dauerhaft nicht zu einer Ermüdung des Kabels und einem späteren Kabelbruch.

Bei einer bevorzugten Ausgestaltung des Intraoralsensors ist das Gehäuse rechteckig mit zwei kürzeren und zwei längeren Stirnseiten ausgebildet. Auf diese Weise läßt sich der Intraoralsensor sowohl für Aufnahmen im Hoch- als auch im Querformat einsetzen.

Mit der Erfindung wird schließlich vorgeschlagen, daß das Anschlußkabel bezüglich der Stirnseiten des Gehäuses unter einem Winkel zwischen 30° und 60° austritt. Auf diese Weise ist für Aufnahmen im Hoch- sowie im Querformat eine gleichermaßen gute und mit geringen Biegungen auskommende Kabelführung gewährleistet.

Weitere Vorteile ergeben sich aus der nachstehenden Beschreibung eines Ausführungsbeispieles. Auf der zugehörigen Zeichnung zeigen:
- Fig. 1: in einer Seitenansicht und teilweise im Schnitt eine Ausführung des erfindungsgemäßen Intraoralsensors;
- Fig. 2: eine Ansicht der Rückseite des Intraoralsensors mit Kabelführung für Aufnahmen im Querformat und
- Fig. 3: eine Ansicht der Rückseite des Intraoralsensors mit Kabelführung für Aufnahmen im Hochformat.

In einem in der Draufsicht etwa rechteckförmigen Gehäuse 1 ist ein in der Figur nicht dargestellter Bildsensor (CCD-Sensor) untergebracht, der in geeigneter Weise mit einer Steuerplatine 2 verbunden ist. An der Steuerplatine 2, die diverse elektronische, nicht näher bezeichnete Bauteile enthält, enden Leitungen 3, die in einem mit 4 bezeichneten Anschlußkabel aus dem Gehäuse herausgeführt und, wie eingangs dargelegt, zu einer (nicht dargestellten) Bildverarbeitungseinheit führen.

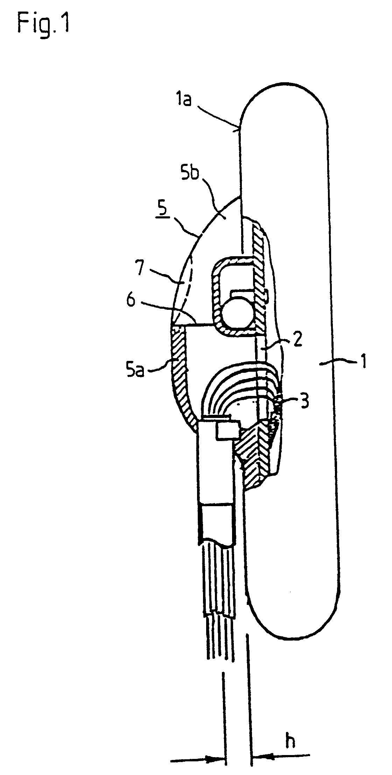

Der Kabelaustritt des Anschlußkabels 4 erfolgt an der Rückwand des Gehäuses 1 unter einem Winkel, der 0° zur Längsebene des Gehäuses 1 liegt. Wie aus Fig. 1 hervorgeht, erstreckt sich beim Ausführungsbeispiel das Anschlußkabel 4 in etwa parallel zur Rückwand 1a des Gehäuses 1, und zwar im Abstand h. Wie aus der Fig. 1 ferner hervorgeht, enthält das Gehäuse 1 im Bereich des Kabelaustritts eine außermittig angeordnete konvexe Wölbung, die durch eine an das Gehäuse angeformte Kappe 5 gebildet ist. Die Kappe 5 ist an der mit 6 bezeichneten Stelle quergeteilt. Im unteren Kappenteil 5a ist das Anschlußkabel 4 zugentlastend gefaßt, wobei das Anschlußkabel 4 und die Leitungsenden wasserdicht mit der Kappe 5 verklebt sind. Beide Kappenteile werden bei der Endmontage ebenfalls wasserdicht miteinander verbunden.

Obgleich die konvexe Wölbung bereits für sich eine gute Positionierhilfe für den Patienten beim Anlegen des Sensors an den aufzunehmenden Zahn ist, kann es doch vorteilhaft sein, zweckmäßigerweise im oberen Kappenteil 5b eine konkav ausgebildete Fingermulde 7, wie sie in Fig. 1 gestrichelt eingezeichnet ist, vorzusehen.

Das Anschlußkabel 4 ist im Bereich seines Austritts aus der Kappe 5 mit einer elastischen Versteifungsmanschette 8 umgeben. Die Versteifungsmanschette 8 verhindert, daß das Anschlußkabel 4 in diesem Bereich zu stark geknickt wird.

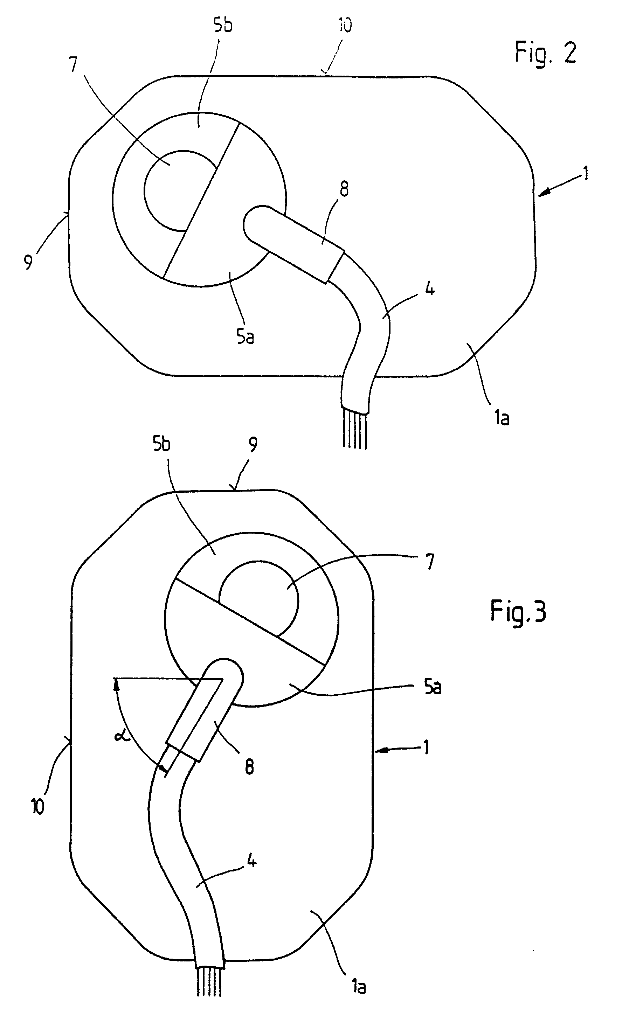

Die Figuren 2 und 3 lassen erkennen, daß das Gehäuse 1 rechteckig mit zwei kürzeren Stirnseiten 9 und zwei längeren Stirnseiten 10 ausgebildet ist. Fig. 2 zeigt die Führung des Anschlußkabels 4 bei Einsatz des Intraoralsensors im Querformat; hingegen zeigt Fig. 3 die Führung des Anschlußkabels 4 bei Verwendung des Intraoralsensors im Hochformat. Um in beiden Fällen das Anschlußkabel 4 nach unten von dem Gehäuse 1 wegführen zu können, tritt das Anschlußkabel 4 bezüglich der Stirnseiten 9,10 des Gehäuses 1 unter einem Winkel a zwischen 30° und 60° aus.

### Bezugszeichenliste

- 1: Gehäuse
- 1a: Rückwand
- 2: Steuerplatine
- 3: Leitung
- 4: Anschlußkabel
- 5: Kappe
- 5a: unteres Kappenteil
- 5b: oberes Kappenteil
- 6: Querteilung
- 7: Fingermulde
- 8: Versteifungsmanschette
- 9: kürzere Stirnseite des Gehäuses
- 10: längere Stirnseite des Gehäuses

- h: Abstand α-Winkel

## Patentansprüche

1. Intraoral eines Patientenmundes plazierbarer Sensor zur Erstellung von Zahn-/Kieferaufnahmen eines Patienten, mit einem schmalen, in der Regel rechteckförmigen Gehäuse (1) zur Aufnahme zumindest eines Bildsensors und einer diesem zugeordneten Steuerplatine (2), an der diverse Leitungen (3) eines Anschlußkabels (4) angeschlossen sind, welches an der Rückwand (1a) des Gehäuses (1) aus dem Gehäuse (1) austritt und mit einer Bildverarbeitungseinheit verbindbar ist,
**dadurch gekennzeichnet,**
daß das Anschlußkabel (4) unter einem Winkel zwischen 0° und 10° zur Ebene der Rückwand (1a) aus dem Gehäuse (1) austritt.

2. Intraoralsensor nach Anspruch 1, dadurch gekennzeichnet, daß das Gehäuse (1) an der Kabelaustrittsstelle mit einer angeformten konvexen Wölbung versehen ist, welche zumindest das Kabelende mit den Leitungsanschlüssen bedeckt.

3. Intraoralsensor nach Anspruch 2, dadurch gekennzeichnet, daß die Wölbung durch eine an das Gehäuse (1) angeformte Kappe (5) gebildet ist, die durch Querteilung (6) zweigeteilt ist, wobei der Kabelaustritt im unteren Teil (5a) der Kappe (5) vorgesehen ist.

4. Intraoralsensor nach Anspruch 3, dadurch gekennzeichnet, daß das Kabelende dichtend mit dem Kappenteil (5a) verklebt ist.

5. Intraoralsensor nach einem der Ansprüche 2 bis 4, dadurch gekennzeichnet, daß die Wölbung mit einer konkaven Fingermulde (7) versehen ist.

6. Intraoralsensor nach einem der Ansprüche 2 bis 5, dadurch gekennzeichnet, daß die Wölbung außerhalb der Mitte der Stirnwand (1a) des Gehäuses (1) angeordnet ist.

7. Intraoralsensor nach einem der vorangehenden Ansprüche, dadurch gekennzeichnet, daß das Anschlußkabel (4) im Bereich des Kabelaustritts von einer elastischen Versteifungsmanschette (8) umgeben ist.

8. Intraoralsensor nach einem der vorangehenden Ansprüche, dadurch gekennzeichnet, daß das Gehäuse (1) rechteckig mit zwei kürzeren (9) und zwei längeren (10) Stirnseiten ausgebildet ist.

9. Intraoralsensor nach Anspruch 8, dadurch gekennzeichnet, daß das Anschlußkabel (4) bezüglich der Stirnseiten (9,10) des Gehäuses (1) unter einem Winkel (α) zwischen 30° und 60° austritt.

## Claims

1. Intraoral sensor which can be placed in a patient's mouth for obtaining tooth/jaw images of the patient, with a narrow, generally rectangular housing (1) for receiving at least one image sensor and, allocated to this housing (1), a control board (2) to which various lines (3) of a connection cable (4) are attached, which connection cable (4) emerges from the housing (1) at the rear wall (1a) of the housing (1) and can be connected to an image processing unit, characterized in that the connection cable (4) emerges from the housing (1) at an angle of between 0° and 10° to the plane of the rear wall (1a).

2. Intraoral sensor according to Claim 1, characterized in that the housing (1) is provided at the cable exit point with an integrally formed convex arch which covers at least the cable end having the line attachments.

3. Intraoral sensor according to Claim 2, characterized in that the arch is formed by a cap (5) which is formed integrally on the housing (1) and which is divided in two by a transverse partition (6), the cable exit being provided in the lower part (5a) of the cap (5).

4. Intraoral sensor according to Claim 3, characterized in that the cable end is bonded tightly to the cap part (5a).

5. Intraoral sensor according to one of Claims 2 to 4, characterized in that the arch is provided with a concave finger depression (7).

6. Intraoral sensor according to one of Claims 2 to 5, characterized in that the arch is arranged off-centre on the end wall (1a) of the housing (1).

7. Intraoral sensor according to one of the preceding claims, characterized in that the connection cable (4), in the area of the cable exit, is surrounded by an elastic stiffening sleeve (8).

8. Intraoral sensor according to one of the preceding claims, characterized in that the housing (1) is of rectangular design, with two shorter (9) and two longer (10) sides.

9. Intraoral sensor according to Claim 8, characterized in that the connection cable (4) emerges at an angle (α) of between 30° and 60° with respect to the sides (9, 10) of the housing (1).

## Revendications

1. Capteur pouvant être placé dans la bouche d'un patient, destiné à obtenir des images des dents et de la mâchoire d'un patient et comportant un boîtier (1) étroit, en général rectangulaire et destiné au logement d'au moins un capteur d'image, et un disque de commande (2) qui est associé à celui-ci et auquel sont raccordées diverses lignes (3) d'un câble de connexion (4) qui sort du boîtier (1) au niveau de la paroi arrière (1a) du boîtier (1) et qui peut être relié à une unité de traitement d'images,
caractérisé en ce que le câble de connexion (4) sort du boîtier (1) sous un angle compris entre 0° et 10° par rapport au plan de la paroi arrière (1a).

2. Capteur intrabuccal selon la revendication 1, caractérisé en ce que le boîtier (1) est muni à l'endroit de sortie de câble d'une voûte formée convexe qui recouvre au moins l'extrémité de câble avec les connexions de lignes.

3. Capteur intrabuccal selon la revendication 2, caractérisé en ce que la voûte est formée par un chapeau (5) qui est formé sur le boîtier (1) et qui est coupé en deux par une coupe transversale (6), la sortie de câble étant prévue dans la partie inférieure (5a) du chapeau (5).

4. Capteur intrabuccal selon la revendication 3, caractérisé en ce que l'extrémité de câble est fixée à la partie de chapeau (5a) par un collage étanche.

5. Capteur intrabuccal selon l'une des revendications 2 à 4, caractérisé en ce que la voûte est munie d'une cavité digitale (7) concave.

6. Capteur intrabuccal selon l'une des revendications 2 à 5, caractérisé en ce que la voûte est agencée en dehors du centre de la paroi frontale (1a) du boîtier (1).

7. Capteur intrabuccal selon l'une des revendications précédentes, caractérisé en ce que le câble de connexion (4) dans la zone de la sortie du câble est entouré d'une manchette de renfort (8) élastique.

8. Capteur intrabuccal selon l'une des revendications précédentes, caractérisé en ce que le boîtier (1) est conçu rectangulaire avec deux côtés frontaux plus courts (9) et deux côtés frontaux plus longs (10).

9. Capteur intrabuccal selon la revendication 8, caractérisé en ce que le câble de connexion (4) sort sous un angle (α) compris entre 30° et 60° par rapport aux côtés frontaux (9, 10) du boîtier (1).
